# EUROPÄISCHE PATENTSCHRIFT

(11) **EP 0 504 107 B1**
(45) Veröffentlichungstag und Bekanntmachung des Hinweises auf die Patenterteilung: **07.12.1994**
(21) Anmeldenummer: 92810161.7
(22) Anmeldetag: 03.03.1992
(51) Int. Cl.: C07C 67/287, C07C 69/63

(54) **Verfahren zur Herstellung von chlorierten Carbonsäureestern**
Process for the preparation of chlorinated esters of carboxylic acids
Procédé de préparation d'esters chlorés d'acides carboxyliques

(30) Priorität: 11.03.1991 CH 715/91
(43) Veröffentlichungstag der Anmeldung: 16.09.1992
(73) Patentinhaber: Säurefabrik Schweizerhall, CH-4133 Schweizerhalle (CH)
(72) Erfinder: Gallegra, Pasquale, Dr., CH-4132 Muttenz (CH); Degischer, Gerhard, Dr., CH-4414 Füllinsdorf (CH)
(74) Vertreter: Schluep, Hans-Peter

(56) Entgegenhaltungen:
- FR-A- 2 269 508
- SU-A- 1 313 850

## Beschreibung

Die Erfindung betrifft ein neuartiges Verfahren zur Herstellung von chlorierten Carbonsäureestern der Formel I 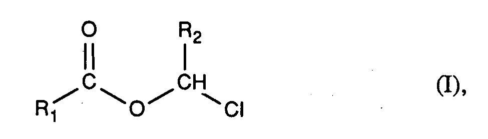
worin R₁ Alkyl, Alkenyl, Alkinyl, Cycloalkyl, Aralkyl oder Aryl bedeutet und R₂ für Wasserstoff, Alkyl oder Cycloalkyl steht, dadurch gekennzeichnet, dass man ein Acylal der Formel II 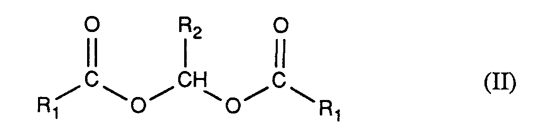
mit Thionylchlorid umsetzt.

Alkyl bedeutet vorzugsweise Niederalkyl, wie geradkettiges oder verzweigtes C₁-C₇-Alkyl, wie Methyl, Ethyl, Propyl, Isopropyl, Butyl, Isobutyl, sec.-Butyl, tert.-Butyl, Pentyl, Isopentyl, sec.-Pentyl, Neopentyl oder eine Hexyl- oder Heptylgruppe, kann aber auch eine C₈-C₁₄-, wie Octyl-, Nonyl- oder Decylgruppe, sein. Als R₁ sind Methyl, Ethyl sowie sekundär-lineare und verzweigte C₃-C₇-Alkylgruppen, wie Isopropyl, But-2-yl, Pent-3-yl, tert.-Butyl oder 2-Methylbut-2-yl, und als R₂ lineare C₁-C₄-Alkylgruppen, wie Methyl, Ethyl, Propyl oder Butyl, bevorzugt.

Alkenyl bedeutet vorzugsweise geradkettiges oder verzweigtes C₂-C₇-Alkenyl, wie Ethenyl, Propenyl, z.B. Allyl, Isopropenyl, Methallyl (Crotyl), Butenyl oder 2-Methylprop-2-enyl.

Alkinyl ist beispielsweise C₃-C₇-Alkinyl, wie Propargyl.

Cycloalkyl ist beispielsweise 3- bis 8-, wie 5- bis 7-gliedriges Cycloalkyl, z.B. Cyclopropyl, Cyclobutyl, Cyclopentyl, Cyclohexyl oder Cycloheptyl.

Aryl ist beispielsweise Phenyl oder Naphthyl; Aralkyl ist beispielsweise Mono-, Di- oder Triphenyl-C₁-C₄-alkyl, wie Benzyl, 1-Phenylethyl, Diphenylmethyl oder Triphenylmethyl. Phenyl und Naphthyl sowie der Phenylteil von Mono-, Di- oder Triphenyl-C₁-C₄-alkyl können unsubstituiert oder durch übliche Substituenten, wie Niederalkyl, z.B. Methyl, Niederalkoxy, z.B. Methoxy, Halogen, z.B. Chlor oder Brom, Trifluormethyl und/oder Nitro substituiert, wie mono-, di- oder trisubstituiert sein, sind jedoch vorzugsweise unsubstituiert.

Sofern nichts anderes angegeben ist, bedeutet der bei der Definition von Resten wie Niederalkyl und Niederalkoxy verwendete Ausdruck "Nieder'', dass die betreffenden Reste bis und mit 7, vorzugsweise bis und mit 4 Kohlenstoffatome enthalten.

Die Verbindungen der Formel I sind wertvolle Zwischenprodukte in der organischen Synthese, insbesondere für die Herstellung von Arzneimittelwirkstoffen. Sie reagieren mit Aminen, Alkoholen und Carbonsäuren unter Einführung der Gruppe der Formel 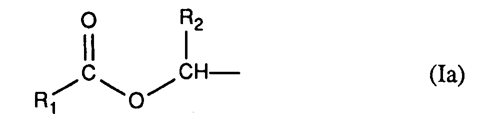
zu den entsprechenden substituierten Aminen, Ethern und Estern. Die Verbindungen der Formel I sind insbesondere geeignet für die Herstellung entsprechender Acyloxyalkylester von β-Lactamantibiotika, wie Penicillinen, beispielsweise von 6-[D-(-)-α-Aminophenylacetamido]-penicillansäure(pivaloyloxy)methylester-hydrochlorid (Pivampicillin), und von Cephalosporinen, beispielsweise von 7-[(2-Amino-4-thiazolyl)-(methoxyiminoacetylamino)-3-methy-8-oxo]- 5-thia- 1-azabicyclo[4.2.0]octencarbonsäure(pivaloyloxy)methylester (Cefetamet pivoxil) und dergleichen.

Die Erfindung betrifft in erster Linie die Herstellung von Verbindungen der Formel I, worin R₁ C₁-C₁₄-Alkyl, C₂-C₇-Alkenyl, C₃-C₇-Alkinyl, 3- bis 8-gliedriges Cycloalkyl, unsubstituiertes oder durch C₁-C₄-Alkyl, C₁-C₄-Alkoxy, Halogen, Trifluormethyl und/oder Nitro substituiertes Phenyl oder Naphthyl oder unsubstituiertes oder im Phenyl durch C₁-C₄-Alkyl, C₁-C₄-Alkoxy, Halogen Trifluormethyl und/oder Nitro substituiertes Mono-, Di- oder Triphenyl-C₁-C₄-alkyl bedeutet und R₂ Wasserstoff, C₁-C₇-Alkyl oder 3- bis 8-gliedriges Cycloalkyl bedeutet.

Die Erfindung betrifft insbesondere die Herstellung von Verbindungen der Formel I, worin R₁ C₁-C₇-Alkyl, wie Methyl oder tert.-Butyl, bedeutet und R₂ für Wasserstoff oder C₁-C₄-Alkyl steht.

Die Erfindung betrifft vorzugsweise die Herstellung von Verbindungen der Formel I, worin R₁ Methyl, Ethyl oder sekundär-lineares oder verzweigtes C₃-C₇-Alkyl, wie Isopropyl oder tert.-Butyl, bedeutet und R₂ für Wasserstoff oder lineares C₁-C₄-Alkyl, wie Methyl oder Ethyl, steht.

Die Erfindung betrifft in allererster Linie die Herstellung von Verbindungen der Formel I, worin R₁ Methyl, Ethyl oder sekundär-lineares oder verzweigtes C₃-C₇-Alkyl, wie Isopropyl oder tert.-Butyl, bedeutet und R₂ für Wasserstoff steht.

Die Erfindung betrifft namentlich die Herstellung der in den Beispielen genannten Verbindungen der Formel I, insbesondere Chlormethylpivalat (R₁ = tert.-Butyl, R₂ = Wasserstoff).

Das übliche Verfahren zur Herstellung von Verbindungen der Formel I ist dadurch gekennzeichnet, dass man ein Säurechlorid der Formel IV 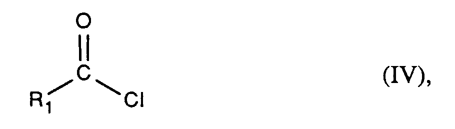
worin R₁ Methyl oder tert.-Butyl bedeutet, in Gegenwart von Zinkchlorid mit Paraformaldehyd kondensiert oder ein annähernd equimolares Gemisch eines entsprechenden Aldehydes der Formel V

R₂―CH=O (V)

und einer Säure der Formel VI 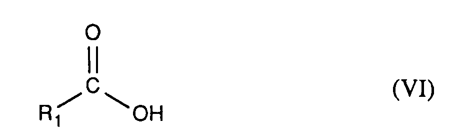
in Gegenwart von Zinkchlorid mit einen Überschuss an Thionylchlorid umsetzt (siehe FR-A 2 269 508). Beide Varianten dieses Verfahrens haben entschiedene Nachteile. Insbesondere ist bekannt, dass das gemäss der erstgenannten Variante erhaltenen Produkt durchwegs durch etwa 10 Mol-% des entsprechenden Bis(α-chloralkyl)ethers verunreinigt ist. Gemäss der zweitgenannten Variante wird, wie das Vergleichbeispiel zeigt, sogar eine erheblich grössere Menge desselben gebildet. Bis(α-chloralkyl)ether werten jedoch auf Grund ihrer, insbesondere bei den niederen Homologen der Reihe sehr hohen, Toxizität erhebliche sicherheitstoxikologische Probleme auf. Dieses unerwünschte Nebenprodukt kann nur sehr schwierig abgetrennt und nur mit grossem Aufwand annähernd vollständig entfernt werden. Ausserdem werden neben Schwefeldioxid und Chlorwasserstoff stets eine Vielzahl weiterer Nebenprodukte gebildet, gemäss der erstgenannten Variante beispielsweise Acylalacetale der Formel VII 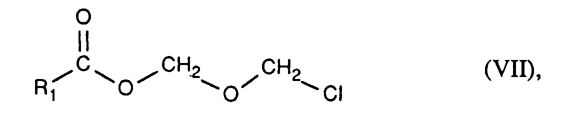
Anhydride der Formel VIII 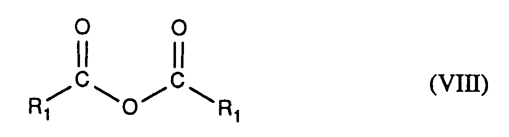
und Acylale der Formel II und gemäss der zweitgenannten Verfahrensvariante Säurechloride der Formel IV. Diese sowie überschüssiger Aldehyd der Formel V und das verwendete Kondensationsmittel erschweren die Isolierung des gewünschten Produktes sehr stark.

Es hat deshalb nicht an Versuchen gefehlt, Verfahren zur Herstellung von Verbindungen der Formel I zu entwickeln, die die genannten Nachteile vermeiden. Die bislang bekannt gewordenen Lösungsvorschläge sind jedoch ebenfalls sicherheitstoxikologisch bedenklich oder zu aufwendig bzw. zu komplex, um industriell anwendbar zu sein.

Beispielsweise wurde vorgeschlagen, die Säure der Formel VI in Form eines Alkalimetallsalzes mit einem entsprechenden Chlorsulfonsäure(α-chlor)alkylester der Formel IX 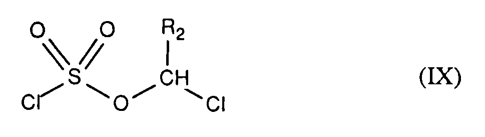
umzusetzen. Jedoch sind Reagentien der Formel IX wenig stabil und werfen auf Grund ihrer hohen Toxizität ihrerseits ihrerseits erhebliche sicherheitstoxikologische Probleme auf, was dem Einsatz dieses Verfahrens im technischen Masstab entgegenstehen.

Einem weiteren Vorschlag zufolge setzt man Methylendiacetat oder Methylendibenzoat (II; R₁= Methyl oder Phenyl, R₂= Wasserstoff) in Gegenwart der 0.2-fachen molaren Menge Aluminiumtrichlorid bei 120° C mit Trimethylchlorsilan um. Das Verfahren ist jedoch für die Herstellung von Verbindungen der Formel I nur sehr bedingt geeignet. So konnte bei der Umsetzung von Methylendibenzoat Chlormethylbenzoat nur in etwa 50 %-iger Ausbeute isoliert werden. Ausgehend vom Methylendiacetat enthielt das Reaktionsgemisch, wie durch Auswertung des ¹H-NMR-Spektrums ermittelt werden konnte, zwar etwa 40 Mol-% Chlormethylacetat, dieses konnte jedoch nicht isoliert werden. Als weiterer Nachteil kommt hinzu, dass stets in equimolarer Menge Gemische von Oligo- und Polysilanolen als unerwünschte, schwerflüchtige Nebenprodukte gebildet werden, die im technischen Masstab nur sehr aufwendig entsorgt werden können. Den bekannten Alternativvorschlägen ist gemeinsam, dass nur speziell ausgewählte, entweder hochtoxische Chlorsulfonsäure(α-chlor)alkylester der Formel IX oder im industriellen Masstab schwer handhabbare (Trimethylchlorsilan-) Reagentien als Chlordonator verwendet werden können. Bei dem zweitgenannten Alternativvorschlag werden infolge der erforderlichen drastischen Reaktionsbedingungen auch nur mässige Ausbeuten erzielt.

Der Erfindung lag dementsprechend die bislang noch nicht gelöste Aufgabe zugrunde, ein Verfahren zur Herstellung von Verbindungen der Formel I zu entwickeln, welches die Nachteile der bekannten Verfahren vermeidet. Diese Aufgabe wird durch das erfindungsgemässe Verfahren sehr gut gelöst.

Das erfindungsgemässe Verfahren beruht auf den im Lichte des Standes der Technik überraschenden Feststellungen, dass Kondensationsmittel, wie Zinkchlorid oder Aluminiumtrichlorid, völlig entbehrlich sind, dass als Chlordonator anstelle hochtoxischer und/oder schwer handhabbarer Spezialragentien Thionylchlorid verwendet werden kann, ohne dass Ausbeute, Umsatzgeschwindigkeit und Produktreinheit beeinträchtigt werden, und dass die Bildung von Bis(α-chloralkyl)ethern sehr weitgehend vermieden werden kann. So ergibt sich aus dem unten gezeigten Ausführungsbeispiel, dass erfindungsgemäss etwa 100-mal weniger Bis(chlormethyl)ether gebildet wird als bei der bereits optimierten bekannten Verfahrensweise gemäss dem Vergleichsbeispiel. Als weiterer Vorteil kommt hinzu, dass die Umsetzung sehr übersichtlich verläuft und weniger Nebenprodukte gebildet werden.

Die Umsetzung von Verbindungen der Formel II mit Thionylchlorid erfolgt üblicherweise unter Verwendung der mindestens equimolaren Menge Thionylchlorid, in An- oder Abwesenheit von Lösungs- oder Verdünnungsmitteln, vorteilhaft bei erhöhter Temperatur und mit anschliessender destillativer Aufarbeitung.

Als Lösungsmittel kommen beispielsweise Halogenalkane oder Halogenaromaten, wie Di-, Tri- oder Tetrachlor-C₁-C₄-alkane, z.B. Methylenchlorid, Trichlorethan oder Chlorbenzol, in Betracht. Vorteilhaft kann die erfindungsgemässe Umsetzung jedoch ohne Lösungsmittel vorgenommen werden, wobei vorteilhaft ein geringer, beispielsweise etwa 1.1-facher bis etwa 2-facher, wie etwa 1.25-facher bis etwa 1.75-facher, insbesondere 1.4-facher bis 1,75-facher, molarer Überschuss an Thionylchlorid über die molare Menge der Verbindung der Formel II verwendet wird.

Die Reaktionstemperatur ist nicht kritisch und kann zwischen etwa 0° C und etwa 150 °C, vorzugsweise von 20 °C bis 150 °C, liegen. Um eine ausreichende Umsatzgeschwindigkeit zu erzielen und optimalen Umsatz innerhalb von etwa 2 bis 12, insbesondere etwa 4 bis bis 8 Stunden zu erzielen, wird jedoch vorteilhaft bei erhöhter Temperatur, vorzugsweise im Temperaturbereich von 40 °C bis 150 °C, beispielsweise von etwa 40 ° C bis etwa 120 °C, wie von etwa 60 °C bis etwa 100 °C, insbesondere von etwa 70 °C bis etwa 80 °C, gearbeitet.

Die destillative Abtrennung des Reaktionsproduktes von überschüssigem Thionylchlorid und als Nebenprodukt gebildetem Säurechlorid der Formel IV erfolgt vorteilhaft unter vermindertem Druck, beispielsweise bei etwa 1 mbar bis etwa 50 mbar, insbesondere bei etwa 10 mbar bis etwa 30 mbar.

In einer bevorzugten Ausführungsform des erfindungsgemässen Verfahrens erwärmt man ein Acylal der Formel II auf etwa 70 °C bis 80 °C, fügt die etwa 1.25-fache bis etwa 1.75-fache molare Menge Thionylchlorid hinzu, lässt etwa 4 bis 8 Stunden reagieren und destilliert dann unter vermindertem Druck, beispielsweise bei etwa 10 mbar bis etwa 30 mbar.

Die Ausgangsverbindungen der Formel II sind bekannt oder werden nach an sich bekannten Verfahren hergestellt.

Beispielsweise können die Acylale (Aldehydacylate) der Formel II hergestellt werden nach einem der in "Houben-Weyl - Methoden der organischen Chemie", E. Müller et al. (Herausgeber), Band 7, Teil 1, 4. Auflage, Georg Thieme Verlag, Stuttgart, S. 442 genannten Verfahren, oder nach Kochhar et al., J. Org. Chem. 48, 1765 (1983), Olah et al., Synthesis p. 962, (1982) oder Michie et al., Synthesis p. 824, (1981).

Die nachfolgen Beispiele dienen zur Illustration der Erfindung; Temperaturen sind in Celsiusgraden, Drucke in mbar angegeben.

Ausführungsbeispiel: 169.9 g Methylendipivalat (II; R₁= tert.-Butyl, R₂= Wasserstoff) werden auf etwa 75 °erwärmt und unter Rühren mit 160.0 g Thionylchlorid versetzt. Man lässt 5 Stunden bei 75° nachrühren. Im Verlaufe der Umsetzung werden insgesamt 45.1 g Schwefeldioxid freigesetzt. In einem Aliquot des Reaktionsgemisches wird die Zusammensetzung des Reaktionsgemisches bestimmt. Die Reaktionsbilanz ist unter Berücksichtigung der separat aufgefangenen flüchtigen Bestandteile folgende:
106.5 g Chlormethylpivalat
45.1 g Schwefeldioxid
85.2g Pivalinsäurechlorid
75.8 g Thionylchlorid
17.0 g Methylendipivalat
0.3 g Bis(chlormethyl)ether
Das Reaktionsgemisch wird dann durch Zugabe von Wasser hydrolysiert und unter vermindertem Druck (etwa 20 mbar) destillativ aufgetrennt; man erhält mindestens 99 %-ig reines Chlormethylpivalat, welches weniger als 0,1 ppm des toxischen Bis(chlormethyl)ethers enthält, in einer Ausbeute von 90 % der Theorie.

### Vergleichsbeispiel:

1.7 g Zinkchlorid werden mit 0,8 ml Wasser verrührt und unter Rühren vorsichtig mit 180.0 g Thionylchlorid versetzt. Man lässt 1 Stunde bei Raumtemperatur nachrühren und fügt dann unter Rühren bei 70° portionsweise eine Suspension von 42.1 g Paraformaldehyd in 85.0 g geschmolzener Pivalinsäure hinzu. Man erwärmt 1 Stunde unter Rühren auf 100°. Im Verlaufe der Reaktion werden insgesamt 36.5 g Chlorwasserstoff und 80 g Schwefeldioxid freigesetzt. In einem Aliquot wird die Zusammensetzung des Reaktionsgemisches bestimmt. Die Reaktionsbilanz ist unter Berücksichtigung der separat aufgefangenen flüchtigen Bestandteile folgende:
106.5 g Chlormethylpivalat
80.0g Schwefeldioxid
13.0 g Pivalinsäurechlorid
30.0 g Thionylchlorid
52.0 g Bis(chlormethyl)ether
36.5 g Chlorwasserstoff
1.7 g Zinkchlorid
Das Reaktionsgemisch wird unter vermindertem Druck (etwa 20 mbar) destillativ aufgetrennt; man erhält etwa 99 %-ig reines Chlormethylpivalat, Ausbeute 85.2 % der Theorie.

## Patentansprüche

1. Verfahren zur Herstellung von chlorierten Carbonsäureestern der Formel I 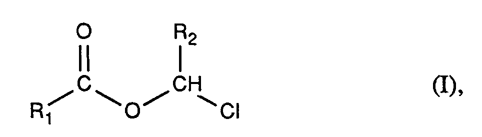 worin R₁ Alkyl, Alkenyl, Alkinyl, Cycloalkyl, Aralkyl oder Aryl bedeutet und R₂ für Wasserstoff, Alkyl oder Cycloalkyl steht, dadurch gekennzeichnet, dass man ein Acylal der Formel II 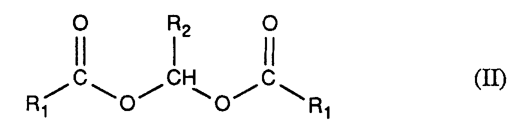 mit Thionylchlorid umsetzt.

2. Verfahren gemäss Anspruch 1, dadurch gekennzeichnet, dass man mit der mindestens equimolaren Menge Thionylchlorid umsetzt.

3. Verfahren gemäss Anspruch 1, dadurch gekennzeichnet, dass man einen 1.1-fachen bis 2-fachen molaren Überschuss an Thionylchlorid über die molare Menge des Acylals der Formel II verwendet.

4. Verfahren gemäss einem der Ansprüche 1 bis 3, dadurch gekennzeichnet, dass man im Temperaturbereich von 40 °C bis 120 °C arbeitet.

5. Verfahren gemäss einem der Ansprüche 1 bis 4, dadurch gekennzeichnet, dass man die destillative Abtrennung des Reaktionsproduktes unter vermindertem Druck vornimmt.

6. Verfahren gemäss einem der Ansprüche 1 bis 5, dadurch gekennzeichnet, dass man ein Acylal der Formel II auf 70° C bis 80° C erwärmt, die 1.25-fache bis 1.75-fache molare Menge Thionylchlorid hinzufügt, 4 bis 8 Stunden reagieren lässt und dann das Reaktionsprodukt unter vermindertem Druck von 10 mbar bis 30 mbar destillativ abtrennt.

7. Verfahren gemäss einem der Ansprüche 1 bis 6, dadurch gekennzeichnet, dass man Verbindungen der Formel I, worin R₁ C₁-C₁₄-Alkyl, C₂-C₇-Alkenyl, C₃-C₇-Alkinyl, 3- bis 8-gliedriges Cycloalkyl, unsubstituiertes oder durch C₁-C₄-Alkyl, C₁-C₄-Alkoxy, Halogen Trifluormethyl und/oder Nitro substituiertes Phenyl oder Naphthyl oder unsubstituiertes oder im Phenyl durch C₁-C₄-Alkyl, C₁-C₄-Alkoxy, Halogen Trifluormethyl und/oder Nitro substituiertes Mono-, Di- oder Triphenyl-C₁-C₄-alkyl bedeutet und R₂ Wasserstoff, C₁-C₇-Alkyl oder 3- bis 8-gliedriges Cycloalkyl bedeutet, herstellt.

8. Verfahren gemäss einem der Ansprüche 1 bis 6, dadurch gekennzeichnet, dass man Verbindungen der Formel I, worin R₁ C₁-C₇-Alkyl bedeutet und R₂ für Wasserstoff oder C₁-C₄-Alkyl steht, herstellt.

9. Verfahren gemäss einem der Ansprüche 1 bis 6, dadurch gekennzeichnet, dass man Verbindungen der Formel I, worin R₁ Methyl, Ethyl oder sekundär-lineares oder verzweigtes C₃-C₇-Alkyl bedeutet und R₂ für Wasserstoff oder lineares C₁-C₄-Alkyl steht, herstellt.

10. Verfahren gemäss einem der Ansprüche 1 bis 6, dadurch gekennzeichnet, dass man Verbindungen der Formel I, worin R₁ Methyl, Ethyl oder sekundär-lineares oder verzweigtes C₃-C₇-Alkyl bedeutet und R₂ für Wasserstoff steht, herstellt.

11. Verfahren gemäss einem der Ansprüche 1 bis 6, dadurch gekennzeichnet, dass man Chlormethylpivalat (der Formel I; R₁ = tert.-Butyl, R₂ = Wasserstoff) herstellt.

12. Verfahren gemäss einem der Ansprüche 1 bis 11, dadurch gekennzeichnet, dass man Chlormethylpivalat (der Formel I; R₁ = tert.-Butyl, R₂ = Wasserstoff) herstellt, indem man Methylendipivalat auf etwa 75 °c erwärmt, mit dem 1,71-fachen molaren Überschuss an Thionylchlorid versetzt, 5 Stunden bei 75 °C nachrührt und das resultierende Reaktionsgemisch durch Zugabe von Wasser hydrolysiert und unter vermindertem Druck von 20 mbar destillativ auftrennt.

## Claims

1. A process for the preparation of a chlorinated carboxylic acid ester of formula I 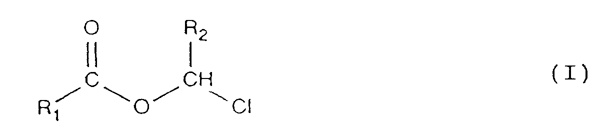 wherein R₁ is alkyl, alkenyl, alkynyl, cycloalkyl, aralkyl or aryl and R₂ is hydrogen, alkyl or cycloalkyl, which process comprises reacting an acylal of formula II 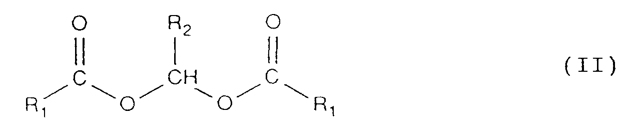 with thionyl chloride.

2. A process according to claim 1 wherein the acylal of formula II is reacted with at least an equimolar amount of thionyl chloride.

3. A process according to claim 1 wherein an excess of 1.1 times to twice the molar amount of thionyl chloride over the acylal of formula II is used.

4. A process according to any one of claims 1 to 3 wherein the reaction is carried out in a temperature range of from 40°C to 120°C.

5. A process according to any one of claims 1 to 4 wherein separation of the reaction product by distillation is carried out under reduced pressure.

6. A process according to any one of claims 1 to 5 wherein an acylal of formula II is heated to from 70°C to 80°C, from 1.25 to 1.75 times the molar amount of thionyl chloride is added, the mixture is allowed to react for from 4 to 8 hours, and then the reaction product is separated by distillation under reduced pressure of from 10 mbar to 30 mbar.

7. A process according to any one of claims 1 to 6 wherein there is prepared a compound of formula I wherein R₁ is C₁-C₁₄alkyl, C₂-C₇alkenyl, C₃-C₇alkynyl or 3- to 8-membered cycloalkyl; or is phenyl or naphthyl each of which is unsubstituted or substituted by C₁-C₄-alkyl, C₁-C₄alkoxy, halogen, trifluoromethyl and/or by nitro, or mono-, di- or tri-phenyl-C₁-C₄alkyl that is unsubstituted or substituted in the phenyl moiety by C₁-C₄alkyl, C₁-C₄alkoxy, halogen, trifluoromethyl and/or by nitro, and R₂ is hydrogen, C₁-C₇alkyl or 3- to 8-membered cycloalkyl.

8. A process according to any one of claims 1 to 6 wherein there is prepared a compound of formula I wherein R₁ is C₁-C₇alkyl and R₂ is hydrogen or C₁-C₄-alkyl.

9. A process according to any one of claims 1 to 6 wherein there is prepared a compound of formula I wherein R₁ is methyl, ethyl or secondary-linear or branched C₃-C₇alkyl, and R₂ is hydrogen or linear C₁-C₄alkyl.

10. A process according to any one of claims 1 to 6 wherein there is prepared a compound of formula I wherein R₁ is methyl, ethyl or secondary-linear or branched C₃-C₇alkyl, and R₂ is hydrogen.

11. A process according to any one of claims 1 to 6 wherein chloromethyl pivalate (of formula I; R₁ = tert-butyl, R₂ = hydrogen) is prepared.

12. A process according to any one of claims 1 to 11 wherein chloromethyl pivalate (of formula I; R₁ = tert-butyl, R₂ = hydrogen) is prepared by heating methylene dipivalate to approximately 75°C, adding a 1.71-fold molar excess of thionyl chloride, stirring at 75°C for 5 hours, and hydrolysing the resulting reaction mixture by the addition of water and separating the reaction mixture by distillation under reduced pressure of 20 mbar.

## Revendications

1. Procédé de préparation d'esters chlorés d'acides carboxyliques de formule I 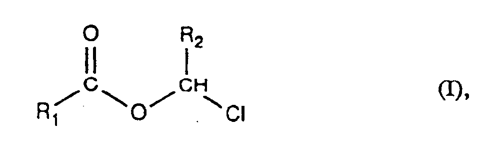 dans laquelle R₁ représente un groupe alkyle, alcényle, alcynyle, cycloalkyle, aralkyle ou aryle et R₂ représente l'hydrogène, un groupe alkyle ou cycloalkyle, caractérisé en ce que l'on fait réagir un acylal de formule II 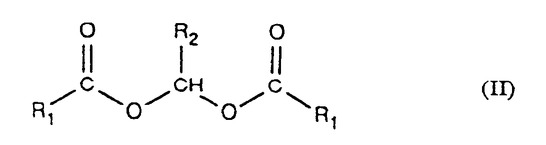 avec le chlorure de thionyle.

2. Procédé selon revendication 1, caractérisé en ce que l'on fait réagir avec le chlorure de thionyle en quantité au moins équimoléculaire.

3. Procédé selon revendication 1, caractérisé en ce que l'on utilise le chlorure de thionyle en quantité 1,1 à 2 fois molaire par rapport à la quantité molaire de l'acylal de formule II.

4. Procédé selon l'une des revendications 1 à 3, caractérisé en ce que l'on opère dans l'intervalle de température de 40 à 120° C.

5. Procédé selon l'une des revendications 1 à 4, caractérisé en ce que l'on sépare le produit de réaction par distillation sous vide.

6. Procédé selon l'une des revendications 1 à 5, caractérisé en ce que l'on chauffe un acylal de formule II à une température de 70 à 80° C, on ajoute du chlorure de thionyle en quantité 1,25 à 1,75 fois la quantité molaire, on laisse réagir pendant 4 à 8 h puis on sépare le produit de réaction par distillation sous un vide de 10 à 30 mbar.

7. Procédé selon l'une des revendications 1 à 6, caractérisé en ce que l'on prépare des composés de formule I dans laquelle R₁ répresente un groupe alkyle en C₁-C₁₄, alcényle en C₂-C₇, alcynyle en C₃-C₇, cycloalkyle de 3 à 8 chaînons, phényle ou naphtyle non substitué ou substitué par des groupes alkyle en C₁-C₄, alcoxy en C₁-C₄, des halogènes des groupes trifluorométhyle et/ou nitro, ou mono-, di- ou triphénylalkyle en C₁-C₄ non substitués ou substitués dans la partie phényle par des groupes alkyle en C₁-C₄, alcoxy en C₁-C₄, des halogènes des groupes trifluorométhyle et/ou nitro, et R₂ représente l'hydrogène un groupe alkyle en C₁-C₇ ou cycloalkyle de 3 à 8 chaînons.

8. Procédé selon l'une des revendications 1 à 6, caractérisé en ce que l'on prépare des composés de formule I dans laquelle R₁ représente un groupe alkyle en C₁-C₇ et R₂ l'hydrogène ou un groupe alkyle en C₁-C₄.

9. Procédé selon l'une des revendications 1 à 6, caractérisé en ce que l'on prépare des composés de formule I dans laquelle R₁ représente un groupe méthyle, éthyle ou un groupe alkyle secondaire linéaire ou ramifié en C₃-C₇, et R₂ représente l'hydrogène ou un groupe alkyle linéaire en C₁-C₄.

10. Procédé selon l'une des revendications 1 à 6, caractérisé en ce que l'on prépare des composés de formule I dans laquelle R₁ représente un groupe méthyle, éthyle ou un groupe alkyle secondaire linéaire ou ramifié en C₃-C₇ et R₂ l'hydrogène.

11. Procéde selon l'une des revendications 1 à 6, caractérisé en ce que l'on prépare le pivalate de chlorométhyle (de formule I dans laquelle R₁ = tert-butyle et R₂ = hydrogène).

12. Procédé selon l'une des revendications 1 à 11, caractérisé en ce que l'on prépare le pivalate de chlorométhyle (de formule I dans laquelle R₁ = tert-butyle et R₂ = hydrogène) en chauffant le dipivalate de méthylène à 75° C environ, en ajoutant le chlorure de thionyle en excès de 1,71 fois la quantité molaire, en agitant pendant encore 5 h à 75° C, en hydrolysant le mélange de réaction par addition d'eau et en le séparant par distillation sous un vide de 20 mbar.
